(19) **Europäisches Patentamt / European Patent Office / Office européen des brevets**

(11) **EP 4 147 634 B1**

(12) # EUROPEAN PATENT SPECIFICATION

(45) Date of publication and mention
of the grant of the patent:
**30.10.2024 Bulletin 2024/44**

(21) Application number: **22194555.3**

(22) Date of filing: **08.09.2022**

(51) International Patent Classification (IPC):
**A61B 5/021** (2006.01)    **A61B 5/00** (2006.01)

(52) Cooperative Patent Classification (CPC):
**A61B 5/021; A61B 5/7207; A61B 5/746**

(54) **PATIENT MONITORING FOR ALARM MANAGEMENT**

PATIENTENÜBERWACHUNG ZUR ALARMVERWALTUNG

SURVEILLANCE DES PATIENTS POUR LA GESTION DES ALARMES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.09.2021 US 202163242698 P**

(43) Date of publication of application:
**15.03.2023 Bulletin 2023/11**

(73) Proprietor: **Welch Allyn, INC.
Skaneateles Falls, NY 13153-0220 (US)**

(72) Inventors:
• **MCSWEENEY, WonKyung
Skaneateles Falls, 13153-0220 (US)**
• **FROIO, Alexandre Hernandez
Skaneateles Falls, 13153-0220 (US)**
• **KELLNER, David L.
Skaneateles Falls, 13153-0220 (US)**
• **KINSLEY, Matthew
Skaneateles Falls, 13153-0220 (US)**
• **QUILTY-KOVAL, Rebecca
Skaneateles Falls, 13153-0220 (US)**
• **ROBERTS, Chris R.
Skaneateles Falls, 13153-0220 (US)**
• **WHITAKER, Tyson B.
Skaneateles Falls, 13153-0220 (US)**

(74) Representative: **Reddie & Grose LLP
The White Chapel Building
10 Whitechapel High Street
London E1 8QS (GB)**

(56) References cited:
**JP-A- 2020 174 690     US-A- 4 050 452
US-A- 4 417 587     US-A1- 2011 077 536
US-A1- 2015 077 268     US-A1- 2015 190 096**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

During continuous patient monitoring, an alarm is often set with a pair of upper and lower alarm limits. The alarm is triggered when a patient's physiological variables are below the lower alarm limit, or when the physiological variables are above the upper alarm limit.

Clinicians are often unable to determine whether an alarm is triggered due to patient deterioration, or due to noise artifacts such as from patient motion. This can lead to confusion regarding the need to respond to the alarm, and can result in alarm fatigue.

US 4050452 A1 discloses an alarm actuating circuit associated with an automatic blood pressure recorder is triggered when a patient's systolic or diastolic pressure does not fall within a prescribed range. The recorder is associated with a pneumatic regulator including a constant-volume reference chamber and associated pressure-sensitive inflation and deflation valves to effect a precise linear depressurization of an inflatable cuff which has been applied to the patient and pressurized to a value higher than the patient's systolic pressure. Pulses obtained from an ultrasonic or other suitable detector sensitive to movements of the patient's arterial wall in synchronism with the blood flow surges as the cuff is depressurized below the patient's systolic pressure are integrated and then translated into variable-duration marking impulses. Such impulses are successively applied to the actuating input of a pen or other marker associated with an X-Y recording chart, the pen being linearly scanned along one chart axis at the constant depressurization rate of the cuff. During such scan, the pen records, on the chart, a linear pattern of marks each of which has a length corresponding to the duration of the marking impulse then applied to its actuating input.

US 4417587 A1 discloses an apparatus for measuring both the systolic and diastolic blood pressure of an individual. A pressurable cuff having an additional tightening band for advantageously positioning a cuff and microphone on an artery is described. The microphone is connected to first the second filters having respective passbands for analyzing the frequency content for recovered pulse signals. The apparatus provides for automatic pressurizing and depressurizing of the cuff whereby the artery is occluded and opened to permit passage of the blood flow. The pressure at the time of receipt of signals from the filters is measured as the systolic pressure. The diastolic blood pressure is measured with apparatus in accordance with the invention by monitoring signals from one of the filters.

The invention is defined in the appended claims.

In general terms, the present disclosure relates to monitoring physiological variables for alarm management. Various aspects are described in this disclosure, which include, but are not limited to, the following aspects.

In one aspect, a method of continuous patient monitoring comprises: initiating an interval mode for measuring blood pressure, the interval mode including measuring the blood pressure at predetermined intervals over a predetermined period of time; taking a first blood pressure measurement at one of the predetermined intervals; determining whether the first blood pressure measurement is abnormal; when the first blood pressure measurement is abnormal, taking a second blood pressure measurement after a predetermined delay; comparing the first and second blood pressure measurements to confirm whether the blood pressure is normal or abnormal; and when the blood pressure is confirmed as abnormal, transmitting the blood pressure to an alarm management application.

In another aspect, a patient monitoring device comprises: at least one processing device; and a memory device storing instructions which, when executed by the at least one processing device, cause the device to: initiate an interval mode for measuring blood pressure at predetermined intervals over a predetermined period of time; take a first blood pressure measurement at one of the predetermined intervals; determine whether the first blood pressure measurement is abnormal; when the first blood pressure measurement is abnormal, take a second blood pressure measurement after a predetermined delay; compare the first and second blood pressure measurements to confirm whether the blood pressure is normal or abnormal; and when the blood pressure is confirmed as abnormal, transmitting the blood pressure to an alarm management application.

In another aspect, a method of patient monitoring for alarm management comprises: inflating a cuff to have a partial pressure around a limb of a patient that is less than a pressure applied to the limb when measuring blood pressure; monitoring an internal pressure inside the cuff; determining motion artifacts from changes in the internal pressure; and transmitting the motion artifacts to delay an alarm triggered by an abnormal measurement received from a physiological sensor attached to the limb.

In another aspect, a patient monitoring device comprises: at least one processing device; and a memory device storing instructions which, when executed by the at least one processing device, cause the device to: inflate a cuff to have a partial pressure around a limb of a patient that is less than a pressure applied to the limb when measuring blood pressure; monitor an internal pressure inside the cuff; determine motion artifacts from changes in the internal pressure; and transmit the motion artifacts to delay an alarm triggered by an abnormal measurement received from a physiological sensor attached to the limb.

In another aspect, a method of patient monitoring for alarm management comprises: receiving electrocardiogram signals from an electrode attached a body; processing the electrocardiogram signals to determine motion artifacts, including calculating motion values that correspond to a strength of the motion artifacts; assigning a location to the motion values based on a location of the electrode on the body; checking for sensors in the location of the motion values, the sensors being used for measuring physiological variables; calculating motion weighted values for physiological measurements obtained from the sensors based on the motion values; and transmitting the motion weighted values to an alarm delay algorithm.

In another aspect, a patient monitoring device comprises: at least one processing device; and a memory device storing instructions which, when executed by the at least one processing device, cause the device to: receive electrocardiogram signals from an electrode attached a body; process the electrocardiogram signals to determine motion values; assign a location to the motion values; check for sensors in the location of the motion values, the sensors being used for measuring physiological variables; calculate motion weighted values for physiological measurements obtained from the sensors based on the motion values; and transmit the motion weighted values to an alarm delay algorithm.

In another aspect, a patient monitoring device comprises: at least one processing device; and a memory device storing instructions which, when executed by the at least one processing device, cause the at least one processing device to: initiate an interval mode that includes measuring blood pressure at predetermined intervals over a predetermined period of time; take a first blood pressure measurement at one of the predetermined intervals; determine whether the first blood pressure measurement is abnormal; take a second blood pressure measurement after a predetermined delay when the first blood pressure measurement is determined abnormal; compare the first and second blood pressure measurements to confirm whether the first blood pressure measurement is abnormal; and trigger an alarm when the first blood pressure measurement is confirmed as abnormal.

In another aspect, a patient monitoring device comprises: at least one processing device; and a memory device storing instructions which, when executed by the at least one processing device, cause the at least one processing device to: inflate a cuff around a limb of a patient to have a partial pressure less than a pressure applied by the cuff to the limb when measuring blood pressure; monitor an internal pressure inside the cuff; identify motion artifacts based on changes in the internal pressure; and delay an alarm triggered by a physiological sensor based on the motion artifacts.

In another aspect, a patient monitoring device comprises: at least one processing device; and a memory device storing instructions which, when executed by the at least one processing device, cause the at least one processing device to: receive electrocardiogram signals from an electrode attached to a body; identify motion artifacts from the electrocardiogram signals; assign a location to the motion artifacts based on where the electrode is attached to the body; identify sensors taking physiological measurements in the location assigned to the motion artifact; calculate motion weighted values based on the motion artifacts for the physiological measurements taken by the sensors; and use the motion weighted values in an alarm delay algorithm.

The invention will now be further described by way of example with reference to the accompanying drawings, in which:

FIG. 1 illustrates an example of a system for monitoring physiological variables of a patient in a clinical environment, the system including a monitoring device and sensors.

FIG. 2 schematically illustrates another example of the system of FIG. 1, which includes the monitoring device and additional sensors for monitoring physiological variables.

FIG. 3 illustrates an example of a method of continuous patient monitoring performed by the monitoring devices of FIGS. 1 and 2.

FIG. 4 illustrates another example of a method of continuous patient monitoring performed by the monitoring devices of FIGS. 1 and 2.

FIG. 5 illustrates another example of a method of continuous patient monitoring performed by the monitoring devices of FIGS. 1 and 2.

FIG. 6 illustrates another example of a method of continuous patient monitoring performed by the monitoring devices of FIGS. 1 and 2.

FIG. 7 illustrates an example of a method of managing alarms performed by the monitoring devices of FIGS. 1 and 2.

FIG. 8 illustrates an example of a method of dynamic ranking of redundant physiological variables performed by the monitoring devices of FIGS. 1 and 2.

FIG. 9 illustrates an example of redundant physiological variables displayed on a display device of the monitoring devices of FIGS. 1 and 2.

FIG. 10 illustrates another example of redundant physiological variables displayed on the display device of the monitoring devices of FIGS. 1 and 2.

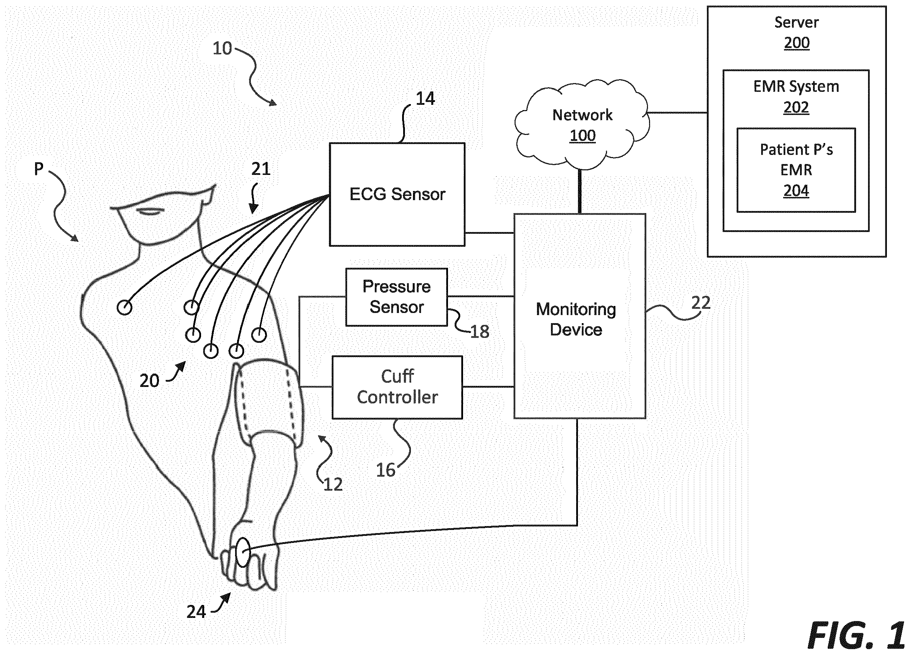

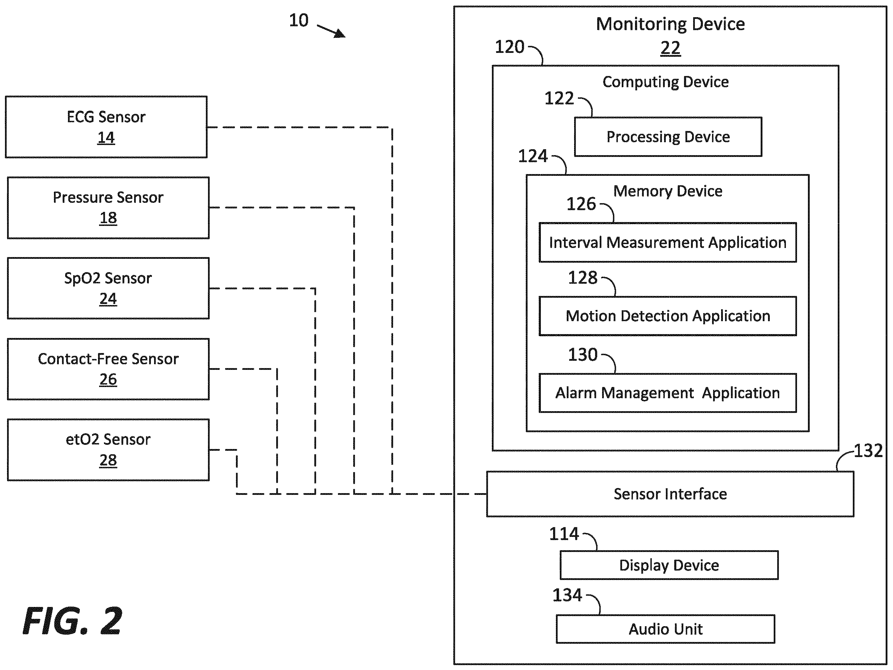

FIG. 1 illustrates an example of a system 10 for monitoring physiological variables of a patient P in a clinical environment such as a hospital. The monitored physiological variables include heart rate, respiration rate, ECG, blood oxygen saturation (SpO2), end tidal carbon dioxide (etCO2), and the like. The system 10 includes sensors connected to a monitoring device 22 for monitoring the physiological variables. Examples of the sensors include an electrocardiogram (ECG) sensor 14, a pressure sensor 18, and a SpO2 sensor 24. The system 10 can also include a contact-free sensor 26 and an etCO2 sensor 28, which are shown in FIG. 2.

As will be described in more detail below, physiological sensing and motion detection functions are combined in one or more of the sensors in the system 10. For example, the pressure sensor 18 can be used to both determine non-invasive blood pressure measurements and detect motion artifacts that can affect or influence the physiological sensing performed by the other sensors in the system 10. In addition, or as an alternative, the ECG sensor 14 can be used to both measure electrocardiogram signals and detect motion artifacts that can affect or influence the physiological sensing performed by the other sensors in the system 10.

The system 10 includes a cuff 12, which is shown placed around an upper arm of the patient P. The cuff 12 is comfortable to wear such that it can be worn by the patient P for a prolonged duration of time such as during twenty-four-hour blood pressure monitoring. The cuff 12 is connected to a cuff controller 16 and a pressure sensor 18, and the cuff controller 16 and the pressure sensor 18 are both connected to the monitoring device 22.

In some examples, the cuff 12, cuff controller 16, pressure sensor 18 are integrated into a single device such as a blood pressure monitor or sphygmomanometer. In some examples, the blood pressure monitor or sphygmomanometer is a separate device that connects to the monitoring device 22. Alternatively, the blood pressure monitor or sphygmomanometer can be integrated with the monitoring device 22 as a single machine.

The cuff controller 16 is used to inflate the cuff 12 until blood flow under the cuff 12 is occluded, and then slowly releases the cuff 12 in a controlled manner to gradually allow blood flow under the cuff 12. The cuff controller 16 can include a pump or similar device to inflate the cuff 12. For example, the cuff controller 16 can inflate the cuff 12 with air to increase the pressure of the cuff 12 around the upper arm of the patient P. The cuff controller 16 can control the amount of pressure applied by cuff 12 around the upper arm of the patient P.

The pressure sensor 18 is used as a non-invasive blood pressure sensor. The pressure sensor 18 detects a first signal from partially occluded blood vessels under the cuff 12, while the pressure of the cuff 12 is slowly released in a controlled manner by the cuff controller 16. The first signal is generated from blood flowing through the partially occluded blood vessels. The pressure sensor 18 can sample the first signal multiple times at various intervals.

The pressure sensor 18 detects a second signal from pressure changes inside the cuff 12 when the cuff is at least partially inflated. The pressure sensor 18 is located within or about the cuff 12 to detect the pressure change inside the cuff. When the cuff 12 is partially inflated, the pressure sensor 18 can detect pressure changes inside the cuff that can result from the patient P shifting their body weight such as when moving from a supine position to a side position or a sitting upright position while resting in bed. Thus, the pressure sensor 18 is also used as a motion sensor to detect motion which can affect the readings of the other physiological variables. This can eliminate the need for a dedicated motion sensor such as an accelerometer attached to the patient P, or piezoelectric sensors, load cells, or combinations thereof that detect movements of the patient P while the patient is supported on a bed or similar support structure.

The pressure sensor 18 sends the first and second signals to the monitoring device 22, which processes the first and second signals to generate outputs. For example, the monitoring device 22 can process the first signal to non-invasively estimate systolic and diastolic blood pressure of the patient P. Also, the monitoring device 22 can process the second signal to detect motion artifacts that can affect or influence the physiological data acquired by the monitoring device 22 from the other sensors in the system 10, such as the physiological variables monitored by the ECG sensor 14, SpO2 sensor 24, contact-free sensor 26, and EtC2 sensor 28.

Thus, the second signal acquired from the pressure sensor 18 can be combined with the data acquired from the other sensors in the system 10 to determine whether an irregular physiological variable reading is caused by motion artifact. For example, when motion artifacts detected from the pressure sensor 18 suggest a movement of the patient P that can cause interference with the electrocardiogram readings from the ECG sensor 14, the monitoring device 22 can instruct the ECG sensor 14 to cancel or retake the electrocardiogram readings, or flag the electrocardiogram readings as likely having an error due to the movement.

As shown in FIG. 1, the ECG sensor 14 includes a plurality of electrodes 20 attached to the body of the patient P. For example, the electrodes 20 can be attached to various locations on the chest, right arm, left arm, right leg, left leg, and head of the patient P.

The electrodes 20 are connected to the ECG sensor 14 by leads 21. The ECG sensor 14 can be a 3/5-lead or 12-lead ECG machine. The signals acquired from the electrodes 20 are used by the ECG sensor 14 for monitoring the electrical activity of the patient P's heart, such as by generating an electrocardiogram that can be displayed on a display device and/or printed.

The ECG sensor 14 also communicates the signals to the monitoring device 22 as raw data that can be used to detect motion artifacts that can affect or influence physiological data acquired from the other sensors in the system 10. The signals from the ECG sensor 14 can be analyzed to determine the location of the motion artifacts. For example, the signals can be sorted based on the location of the electrodes such that the signals can be used to detect motion artifacts on the right arm, left arm, right leg, left leg, and the like. Thus, the ECG sensor 14 can be used as a motion sensor. This can eliminate the need for a dedicated motion sensor such as an accelerometer attached to the patient P, or piezoelectric sensors, load cells, or combinations thereof that detect movements of the patient while supported on a bed or similar structure.

The motion artifacts detected from the signals acquired from the ECG sensor 14 are used by the monitoring device 22 to improve the physiological data acquired from the other sensors in the system 10. For example, when the signal from the ECG sensor 14 detects patient motion that can interfere with a blood pressure measurement from the pressure sensor 18, the monitoring device 22 can cancel or retake the blood pressure measurement, or flag the blood pressure measurement as likely having an error due to the motion. Thus, the signal acquired from the ECG sensor 14 can be combined with physiological data from the other sensors in the system 10 to determine whether an irregular physiological variable reading is caused by motion.

As further shown in FIG. 1, the SpO2 sensor 24 is a clip that is attached to a finger of the patient P. Alternatively, the SpO2 sensor 24 can attach to other body parts of the patient P such as the patient's earlobe. The SpO2 sensor 24 is connected to the monitoring device 22, and sends data to the monitoring device 22 to estimate the blood oxygen saturation of the patient P.

As shown in FIG. 1, the monitoring device 22 communicates with a server 200 via a communications network 100. The server 200 operates to manage the patient P's medical history and information. The server 200 can be operated by a healthcare service provider, such as a hospital or medical clinic. The monitoring device 22 sends physiological data acquired from the sensors in the system 10 to the server 200 via the connection to the communications network 100. In at least some examples, the server 200 is a cloud server or similar type of server.

The server 200 can include an electronic medical record (EMR) system 202 (alternatively termed electronic health record (EHR)). Advantageously, the server 200 can store the physiological data acquired from the sensors in the system 10 in an electronic medical record (EMR) 204 or electronic health record of the patient P located in the EMR system 202 via the connection with the monitoring device 22 over the communications network 100.

The communications network 100 communicates data between one or more devices, such as between the monitoring device 22 and the server 200. The communications network 100 may also be used to communicate data between the one or more sensors and devices in the system 10 such as between the ECG sensor 14, cuff controller 16, pressure sensor 18, and SpO2 sensor 24, and also the contact-free sensor 26 and etCO2 sensor 28, which are shown in FIG. 2.

The communications network 100 can include any type of wired or wireless connections, or any combinations thereof. Examples of wireless connections include cellular network connections such as 4G or 5G. The wireless connections can also be accomplished using Wi-Fi, ultra-wideband (UWB), Bluetooth, radio frequency identification (RFID), and the like.

FIG. 2 schematically illustrates another example of the system 10, which includes the monitoring device 22 and sensors for monitoring physiological variables of the patient. In this example, in addition to the ECG sensor 14, pressure sensor 18, and SpO2 sensor 24, a contact-free sensor 26 and an etCO2 sensor 28 are also connected to the monitoring device 22.

The contact-free sensor 26 is a sensor that can continuously monitor physiological variables of the patient without physically contacting the patient. For example, the contact-free sensor 26 can be positioned on a bed frame below a mattress on which the patient rests in the clinician environment. The contact-free sensor 26 can be used to measure physiological variables such as heart rate and respiration rate of the patient. An example of the contact-free sensor 26 is described in U.S. Patent No. 9,775,758.

The etCO2 sensor 28 is a sensor that measures the level of carbon dioxide that is released at the end of an exhaled breath, called end-tidal carbon dioxide (etCO2). The etCO2 sensor 28 can also be used to measure the respiration rate of the patient. The etCO2 sensor 28 can be attached to a breathing tube, a face mask, or similar breathing devices.

As shown in FIG. 2, the monitoring device 22 includes a computing device 120 with at least one processing device 122 and a memory device 124. The at least one processing device 122 is an example of a processing unit such as a central processing unit (CPU). In some examples, the at least one processing device 122 can include one or more digital signal processors, field-programmable gate arrays, or other electronic circuits.

The memory device 124 operates to store data and instructions for execution by the at least one processing device 122, including an interval measurement application 126, a motion detection application 128, and an alarm management application 130, described in more detail below. The memory device 124 includes computer-readable media, which may include any media that can be accessed by the monitoring device 22. By way of example, computer-readable media include computer readable storage media and computer readable communication media.

Computer readable storage media includes volatile and nonvolatile, removable and non-removable media implemented in any device configured to store information such as computer readable instructions, data structures, program modules, or other data. Computer readable storage media can include, but is not limited to, random access memory, read only memory, electrically erasable programmable read only memory, flash memory, and other memory technology, including any medium that can be used to store information that can be accessed by the monitoring device 22. The computer readable storage media is non-transitory.

Computer readable communication media embodies computer readable instructions, data structures, program modules or other data in a modulated data signal such as a carrier wave or other transport mechanism and includes any information delivery media. The term "modulated data signal" refers to a signal that has one or more of its characteristics set or changed in such a manner as to encode information in the signal. By way of example, computer readable communication media includes wired media such as a wired network or direct-wired connection, and wireless media such as acoustic, radio frequency, infrared, and other wireless media. Combinations of any of the above are within the scope of computer readable media.

The interval measurement application 126 is used by the monitoring device 22 to perform an interval mode for measuring physiological variables. For example, the cuff controller 16, pressure sensor 18, and monitoring device 22 can operate under the interval mode where blood pressure readings are taken at predetermined intervals over a predetermined period of time. The cuff 12 is continuously worn by the patient P, and the interval measurement application 126 instructs the cuff controller 16 to inflate the cuff 12 at each interval, while using the pressure sensor 18 to non-invasively estimate systolic and diastolic blood pressure of the patient P when the pressure of the cuff 12 is slowly released in a controlled manner by the cuff controller 16. As an illustrative example, the interval mode can occur over a 24 hour period of time, and each interval can occur every 20 or 30 minutes during the 24 hour period.

The motion detection application 128 detects patient motion using one or more of the sensors connected to the monitoring device 22. The motion detection application 128 can detect patient motion using the cuff 12, cuff controller 16, and pressure sensor 18. For example, the motion detection application 128 can instruct the cuff controller 16 to inflate the cuff 12 to maintain a partial pressure around a limb (e.g., arm) of the patient P. The partial pressure is a pressure that allows patient motion to be detected from internal pressure changes inside the cuff 12 that can result from the patient P shifting their body weight while resting on a surface such as a bed. The pressure changes inside the cuff 12 are detected by the pressure sensor 18.

The partial pressure is less than the pressure that is applied to the patient P's arm when measuring the blood pressure of the patient P. Additionally, the partial pressure is less than the safe venous return pressure (SVRP), which is a standard for patient safety that specifies a pressure limit at which reasonable venous return can take place to prevent excessive blood pooling. For adults, the SVRP is 15mmHg. For neonates, the SVRP is 5 mmHg.

In one example, the cuff 12 is inflated to maintain the partial pressure between the intervals in the interval mode. In another example, the cuff 12 is inflated to maintain the partial pressure for a continuous period of time when the monitoring device 22 is not operating under the interval mode. In another example, the cuff 12 is inflated to maintain the partial pressure when an abnormal reading is received from one of the other sensors connected to the monitoring device 22, during continuous monitoring physiological variables including heart rate, respiration rate, blood oxygen saturation (SpO2), end tidal carbon dioxide (etCO2), and the like.

The motion detection application 128 can detect patient motion using the signals from the ECG sensor 14. For example, the motion detection application 128 can instruct the at least one processing device 122 to analyze the signals received by the ECG sensor 14 using one or more filters to detect motion. Examples of the one or more filters for filtering the signals from the ECG sensor 14 can include band-pass, band-stop, high-pass, and low-pass filters.

The alarm management application 130 can automatically cancel, delay, and/or reset one or more alarms that are triggered (e.g., due to one or more physiological variables being above upper alarm limits or below lower alarm limits) when patient motion is detected from the motion detection application 128. Advantageously, the alarm management application 130 can eliminate the need for a clinician to manually cancel, delay, and/or reset the alarms.

The alarm management application 130 can also provide a selectable time delay for alarm events (e.g., when an abnormal blood pressure measurement is received in the interval mode) that a clinician can adjust. For example, a default time delay can be set at two minutes after an alarm is triggered, and a clinician can adjust the time delay to have a 10 minute delay via a user interface displayed on a display device 114 of the monitoring device 22. The time delay allows a clinician to finish a task in another room when the alarm is not a threat to the patient.

The monitoring device 22 further includes a sensor interface 132 that operates to communicate with the various sensors of the system 10. The sensor interface 132 can include both wired interfaces and wireless interfaces. The ECG sensor 14, pressure sensor 18, SpO2 sensor 24, contact-free sensor 26, and etCO2 sensor 28 can wirelessly connect to the sensor interface 132 through Wi-Fi, ultra-wideband (UWB), Bluetooth, and similar types of wireless connections. Alternatively, or in addition to wireless connectivity, the ECG sensor 14, pressure sensor 18, SpO2 sensor 24, contact-free sensor 26, and etCO2 sensor 28 can also connect to the monitoring device 22 using wired connections that plug into the sensor interface 132.

As shown in FIG. 2, the monitoring device 22 includes the display device 114, which operates to display one or more user interfaces. In some examples, the display device 114 is a touchscreen such that the user interfaces operate to receive inputs from a clinician. In such examples, the display device 114 operates as both a display device and a user input device. The monitoring device 22 can also support physical buttons on a housing of the device that operate to receive inputs from the clinician to control operation of the monitor device and enter data.

The monitoring device 22 can also include an audio unit 134. The audio unit 134 generates audio sounds such as to sound an alarm when an alarm is triggered by the alarm management application 130. Also, the audio unit 134 can be used to provide instructions.

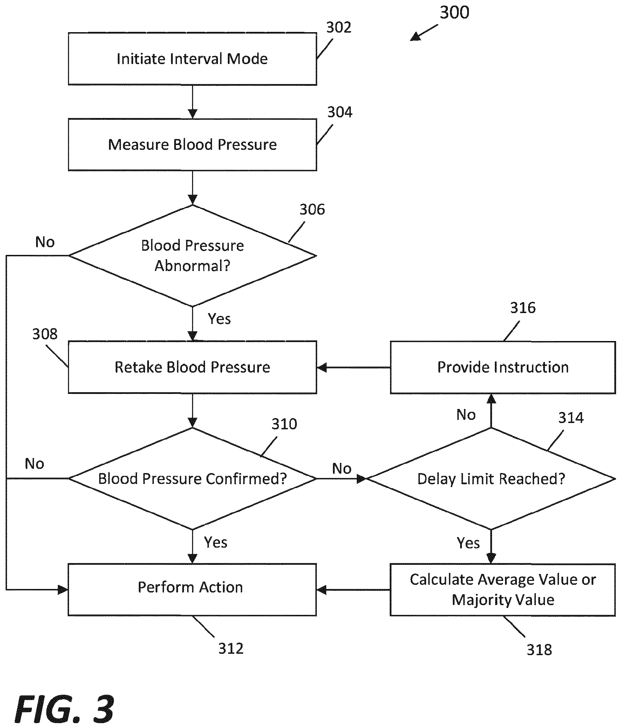

FIG. 3 illustrates an example of a method 300 of continuous patient monitoring. The method 300 can be performed by the interval measurement application 126 using the cuff 12, cuff controller 16, and pressure sensor 18 to retake non-invasive blood pressure measurements during the interval mode when an abnormal blood pressure measurement is detected.

The method 300 overcomes obstacles in the interval mode where the cuff 12 stays on the patient's arm over a predetermined period of time (e.g., 24 hours), and the blood pressure measurements are automatically triggered at predetermined intervals (e.g., every 20 or 30 minutes). In the interval mode, blood pressure measurements are typically reported without a clinician being present such that a clinician is unable to rerun the blood pressure measurement or perform a manual blood pressure reading to confirm whether an abnormal blood pressure measurement is true or accurate. This can lead to inaccurate blood pressure measurements being recorded by the monitoring device 22 and stored in the EMR 204 of the patient. Additionally, this can trigger false alarms and can lead to alarm fatigue.

An abnormal blood pressure measurement is above an upper alarm limit or below a lower alarm limit. The upper and lower alarm limits are based on normal or default values. A normal resting blood pressure for human adults is approximately 120/80 mmHg, a high blood pressure for human adults is considered to be 140/90 mmHg or higher, and a low blood pressure for human adults is considered to be 90/60 mmHg or lower. Thus, an abnormal blood pressure measurement is above 140/90 mmHg or below 90/60 mmHg. Additionally, an abnormal blood pressure measurement is when no blood pressure measurement is obtained due to technical error.

The method 300 includes an operation 302 of initiating the interval mode for measuring blood pressure. In the interval mode, the cuff 12 stays on the patient's arm over a predetermined period of time (e.g., 24 hours), and the blood pressure measurements are automatically triggered at predetermined intervals (e.g., every 20 or 30 minutes).

Next, the method 300 includes an operation 304 of taking a blood pressure measurement in accordance with the interval mode. The blood pressure measurement taken at operation 304 is referred to herein as a first blood pressure measurement. The blood pressure measurement is taken by using the cuff controller 16 to inflate the cuff 12 until blood flow under the cuff 12 is occluded, and then slowly releasing the cuff 12 in a controlled manner to gradually allow blood flow under the cuff 12 while using the pressure sensor 18 to detect a signal from the partially occluded blood vessels under the cuff 12 for non-invasively estimating systolic and diastolic blood pressure. The blood pressure measurement is automatically measured at operation 304 without assistance from the patient, a clinician, or other personnel around the patient.

Next, the method 300 includes an operation 306 of determining whether the blood pressure measurement obtained from operation 304 is abnormal (i.e., is outside of an upper or lower alarm limit, or not obtained due to technical error). When the blood pressure measurement is not abnormal such that it is within a normal range (i.e., "No" at operation 306), the method 300 proceeds to an operation 312 of transmitting the blood pressure measurement.

When the blood pressure measurement obtained from operation 304 is outside the upper and lower alarm limits or no blood pressure measurement is obtained due to technical error such that the blood pressure measurement is abnormal (i.e., "Yes" at operation 306), the method 300 proceeds to an operation 308 of retaking the blood pressure measurement. The blood pressure measurement retaken at operation 308 is referred to herein in some instances as a second blood pressure measurement or as a third blood pressure measurement.

Operation 308 is performed after a predetermined delay, which is an amount of time after the blood pressure measurement initially taken in operation 304. For example, the blood pressure is retaken at operation 308 at least 30 seconds after the blood pressure is initially taken at operation 304. This avoids prolonged periods in which the cuff 12 is continuously inflated or repeatedly inflated, which can affect blood circulation and cause blood pooling. The predetermined amount of time between operations 304, 308 can be based on a regulatory standard for a long term automatic mode of measuring blood pressure.

Next, the method 300 proceeds to operation 310 of comparing the second blood pressure measurement retaken at operation 308 to the first blood pressure measurement taken at operation 304 to confirm whether the blood pressure of the patient is normal or abnormal. In some examples, confirmation at operation 310 is based on whether the first and second blood pressure measurements are both abnormal. In other examples, confirmation at operation 310 is based on whether the second blood pressure measurement matches the first blood pressure measurement or is within a predefined threshold of the first blood pressure measurement.

When the blood pressure is confirmed as abnormal (i.e., "Yes" at operation 310), the method 300 proceeds to the operation 312 of performing an action based on the confirmed abnormal blood pressure measurement. Operation 312 can include triggering an alarm during the interval mode such as by communicating the confirmed abnormal blood pressure measurement to the alarm management application 130. Additionally, or alternatively, operation 312 can include storing the blood pressure in the EMR 204 via the communications network 100.

In examples when the blood pressure is confirmed as normal (i.e., "No" at operation 310), the method 300 includes storing the second blood pressure measurement retaken at operation 308 in the EMR 204 via the communications network 100.

In some examples, when the blood pressure is not confirmed as abnormal (i.e., "No" at operation 310), the method 300 proceeds to operation 314 of determining whether a delay limit is reached. The delay limit is based on an allowed delay for the interval blood pressure measurements. The delay limit can be a default value or can be value set by a clinician based on the condition of the patient. For example, when the patient is healthy, the delay limit can allow for a longer delay because there is less urgency to receive the blood pressure measurement. However, when the patient is in a deteriorated state such as due to sepsis, the delay limit is shorter because there is greater urgency to receive the blood pressure measurement.

When the delay limit is not reached (i.e., "No" at operation 314), the method 300 can optionally proceed to operation 316 of providing an instruction to the patient. As an illustrative example, the abnormal blood pressure measurement can be due to patient motion which either causes the blood pressure measurement to be incorrect, or causes there to be no blood pressure measurement due to technical error. In some instances, the method 300 can include receiving data indicating that the patient is moving from another sensor in the system 10 such as the ECG sensor 14. In such examples, the instruction provided in operation 316 can be for the patient to remain still or stop moving to retake the blood pressure measurement without motion artifacts.

In some examples, the instruction provided in operation 316 is an audio message that is played back by the audio unit 134 of the monitoring device 22. Alternatively, or in addition to the audio message, the instruction provided in operation 316 can be a message displayed on the display device 114, or can be a blinking light understood by the patient as an instruction to remain still while their blood pressure is being retaken by the blood pressure monitor.

Next, the method 300 repeats the operation 308 of retaking the blood pressure measurement. Operation 308 is performed after the predetermined delay (e.g., 30 seconds) has passed from when the blood pressure measurement was previously retaken in operation 308.

The method 300 repeats the operation 310, this time comparing the third blood pressure measurements retaken at operation 308 to the first blood pressure measurement taken at operation 304 to confirm whether the blood pressure is normal or abnormal. When the blood pressure is confirmed as abnormal (i.e., "Yes" at operation 310), the method 300 proceeds to operation 312 of transmitting a confirmed blood pressure measurement which can include the first blood pressure measurement, or the second blood pressure measure, or the third blood pressure measurement, or an average of the first, second, and third blood pressure measurements.

In some examples, when the blood pressure is confirmed as normal (i.e., "No" at operation 310), the method 300 includes transmitting the third blood pressure measurement retaken at operation 308 to the server 200 via the communications network 100 for storage in the EMR 204. Alternatively, when the blood pressure is not confirmed (i.e., "No" at operation 310), the method proceeds again to operation 314 of determining whether the delay limit is reached.

When the delay limit is reached (i.e., "Yes" at operation 314), the method 300 proceeds to operation 318 which can include determining an average value of the blood pressure measurement taken at operation 304 and the blood pressure measurement(s) retaken at operation 308. Alternatively, or additionally, operation 318 can include determining a majority value (i.e., a blood pressure measurement that appears most often, i.e., a mode value) between the blood pressure measurement taken at operation 304 and the blood pressure measurement(s) retaken at operation 308. Operation 312 can include sending at least one of the average value and the majority value of the blood pressure measurements taken in operations 304, 308.

At operation 312, the method 300 can include sending the blood pressure measurement to the server 200 for storage in the EMR 204 of the patient located in the EMR system 202. Additionally, the alarm management application 130 can acquire the blood pressure measurement sent at operation 312 to trigger an alarm when the measurement is abnormal. Advantageously, the method 300 can reduce false alarms while the monitoring device 22 operates under the interval mode for measuring blood pressure.

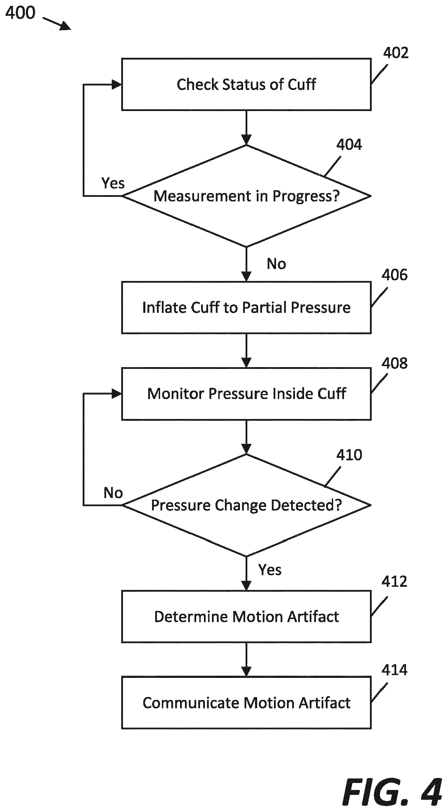

FIG. 4 illustrates another example of a method 400 of continuous patient monitoring. The method 400 is performed to detect patient motion during continuous physiological variable monitoring. The method 400 can be performed by the motion detection application 128 installed on the monitoring device 22 using the cuff 12, cuff controller 16, and pressure sensor 18.

The method 400 can be performed to detect patient motion during an interval mode for measuring physiological variables performed by the interval measurement application 126. In some instances, the method 400 is performed to detect patient motion between measurement intervals for systolic and diastolic blood pressure. Thus, the method 400 can be performed to monitor for patient motion whenever the cuff 12 is not being used to measure patient blood pressure. In the method 400, the cuff 12 can be used as a continuous motion sensor.

The patient motion can affect or influence the signals received by the monitoring device 22 from the other physiological sensors in the system 10, causing erroneous readings. Additionally, the patient motion can naturally cause some of the physiological variables to change such as heart rate and respiration rate. Advantageously, the patient motion detected in accordance with the method 400 can be used by the alarm management application 130 installed on the monitoring device 22 to cancel, delay, or reset an alarm triggered by an abnormal reading received by one of the other sensors connected to the monitoring device 22.

The method 400 includes an operation 402 of checking a status of the cuff 12 to determine whether the cuff 12 is being used to measure blood pressure or not. For example, the cuff 12 can be used during the interval mode to measure blood pressure at predetermined intervals (e.g., 30 mins) over a predetermined period of time (e.g., 24 hours). When the cuff 12 is being used to measure blood pressure, (i.e., "Yes" at operation 404) the method 400 returns to operation 402 of checking a status for a blood pressure measurement. When the blood pressure measurement is not in progress (i.e., "No" at operation 404) the method 400 proceeds to operation 406 of instructing the cuff controller 16 to partially inflate the cuff 12.

At operation 406, the motion detection application 128 instructs the cuff controller 16 to inflate the cuff 12 to have a partial pressure that is sufficient for the pressure sensor 18 (which is located within or about the cuff 12) to detect internal pressure changes inside the cuff 12. The partial pressure allows the pressure sensor 18 to detect internal pressure changes inside the cuff 12 that can result from patient motion such as when the patient shifts their body weight when supported on a surface as a bed to move from a supine position to a sitting upright position.

At operation 406, the motion detection application 128 instructs the cuff controller 16 to inflate the cuff 12 to have the partial pressure, which is less than the pressure applied to the patient's arm when estimating the blood pressure of the patient. Also, the partial pressure is less than the SVRP (e.g., less than 15mmHg for adults, and less than 5 mmHg for neonates).

Next, the method 400 proceeds to operation 408 of monitoring an internal pressure inside the cuff 12 to determine whether there are any pressure changes inside the cuff 12. As described above, pressure changes inside the cuff 12 may result of the patient shifting their body weight while supported on a surface such as a bed, and can be used to detect patient motion.

When no pressure change is detected (i.e., "No" at operation 410), the method 400 returns to operation 408 and continues monitoring the internal pressure inside the cuff 12. When a pressure change is detected (i.e., "Yes" at operation 410), the method 400 proceeds to operation 412 of processing the pressure change inside the cuff 12 to determine a motion artifact.

Operation 412 includes processing raw data obtained from the pressure sensor 18 to determine a motion artifact. In some examples, the raw data is processed to calculate a motion value that quantifies a strength or intensity of the motion artifact. The processing at operation 412 can also include normalizing the motion value to be on a common scale.

The processing of the raw data in operation 412 can also include assigning a location to the motion artifact. For example, the motion artifact can be assigned the location of the arm on which the cuff 12 is attached. The location of the motion artifact can be used by the alarm management application 130 to determine an affect or influence on physiological variables measured by other sensors in the same location, such at the blood oxygen saturation measured from the SpO2 sensor 24 when attached to the same arm as the cuff 12.

Next, the method 400 proceeds to operation 414 of communicating the motion artifact and assigned location to the alarm management application 130. The motion artifact can be used as an input for an algorithm performed by the alarm management application 130 to cancel, delay, and/or reset one or more alarms triggered by an abnormal reading from another sensor in the system 10. Additionally, the motion artifact can be used to determine a dynamic ranking of redundant physiological variables, which will be described in more detail below.

Also, the motion artifact can be used by the monitoring device 22 to improve blood pressure measurements taken during the interval mode. For example, when the motion artifact is detected within a predetermine amount of time (e.g., 30 seconds) before a scheduled blood pressure measurement in accordance with the interval mode, the monitoring device 22 can issue a warning or an instruction for the patient to remain still before the schedule measurement.

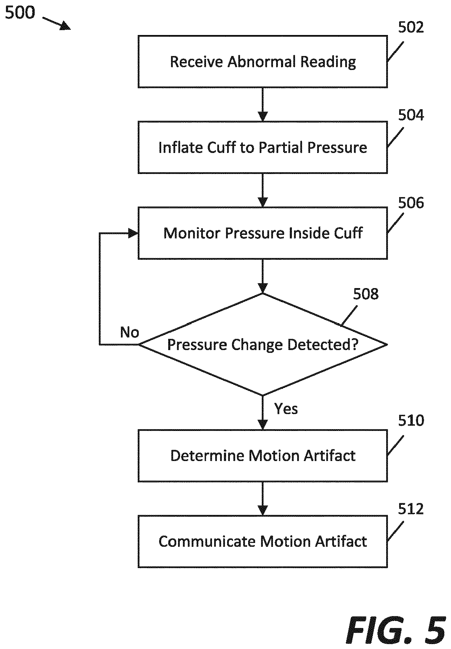

FIG. 5 illustrates another example of a method 500 of continuous patient monitoring. The method 500 can be performed by the motion detection application 128 installed on the monitoring device 22 using the cuff 12, cuff controller 16, and pressure sensor 18. The method 500 includes many of the operations included in the method 400.

The method 500 differs from the method 400 in that the method 500 includes an operation 502 of receiving an abnormal reading from another sensor in the system 10 that triggers the method 500 perform operations 504-514 using the cuff 12, cuff controller 16, and pressure sensor 18 to detect patient motion. This information can be used by the alarm management application 130 to cancel, delay, and/or reset one or more alarms triggered by the abnormal reading. Thus, the method 500 is performed when an abnormal reading is received from another sensor in the system 10 such that the cuff 12 is used as an event driven motion sensor. This is different from the method 400 which is performed to continuously monitor for patient motion whenever the cuff 12 is not being used to measure patient blood pressure.

As an illustrative example, operation 502 can include receiving an abnormal SpO2 reading from the SpO2 sensor 24 attached to the same arm of the patient as the cuff 12 (see FIG. 1). When the abnormal SpO2 reading is received, the method 500 performs operations 504-514 using the cuff 12, cuff controller 16, and pressure sensor 18 to determine whether there are patient motion artifacts on the arm of the patient where the SpO2 sensor 24 is attached. Thus, the method 500 is performed to verify whether an abnormal reading from the SpO2 sensor 24 is due to patient motion or not, and this information is used by the alarm management application 130 to cancel, delay, and/or reset one or more alarms triggered by the abnormal SpO2 reading.

After receiving the abnormal reading in operation 502, the method 500 includes an operation 504 of instructing the cuff controller 16 to partially inflate the cuff 12 (see description of operation 406 in the method 400); an operation 506 of monitoring the pressure inside the cuff 12 (see description of operation 408 in the method 400); an operation 508 of determining whether a pressure change is detected inside the cuff 12 (see description of operation 410 in the method 400); an operation 510 of determining a motion value (see description of operation 412 in the method 400); and an operation 512 of communicating the motion artifact to the alarm management application 130 (see description of operation 414 in the method 400).

Like in the method 400, the motion artifact communicated in operation 512 can be used as an input for an algorithm performed by the alarm management application 130. Also, the motion artifact communicated in operation 512 can be used to determine a dynamic ranking of redundant physiological variables, which will be described in more detail below.

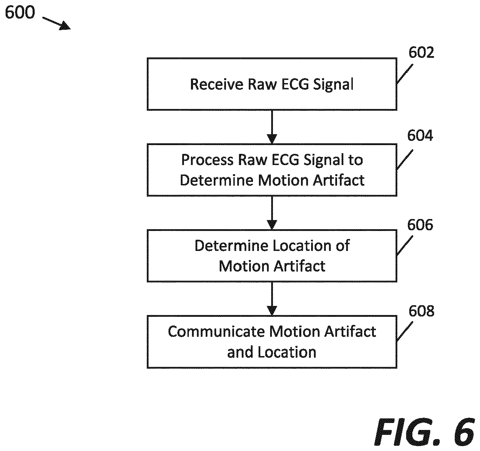

FIG. 6 illustrates another example of a method 600 of continuous patient monitoring. The method 600 is performed by the motion detection application 128 installed on the monitoring device 22 using the ECG sensor 14. The method 600 is performed to detect patient motion from raw ECG signals acquired from the electrodes 20 of the ECG sensor 14.

During continuous patient monitoring, physiological sensors are easily affected by patient motion artifacts. For example, each sensor in the system 10 can be affected individually or in combination with other sensors in the system 10 based on the strength and location of the patient motion artifact. As will be described in more detail, the method 600 analyzes the raw ECG signals from the ECG sensor 14 to determine the strength and location of patient motion artifacts, which can be used by the alarm management application 130 to cancel, delay, and/or reset one or more alarms triggered during continuous physiological variable monitoring.

Referring now to FIG. 6, the method 600 includes an operation 602 of receiving raw ECG signals from the ECG sensor 14. As described above, the raw ECG signals are detected by the electrodes 20 attached to the body of the patient, and are communicated to the ECG sensor 14 via the leads 21. The ECG sensor 14 can then send the raw ECG signals to the monitoring device 22. In alternative examples, the monitoring device 22 can receive the raw ECG signals directly from the electrodes 20 when the leads 21 are directly connected to the monitoring device 22.

Next, the method 600 includes an operation 604 of processing the raw ECG signals to determine a motion artifact. The motion artifact is determined from signal noise in the raw ECG signals. The signal noise can be filtered to improve detection of motion artifacts. For example, low frequency noise is used to identify motion artifacts, while high frequency noise is excluded.

In some examples, the processing performed at operation 604 can include calculating a motion value that corresponds to a strength or magnitude of the motion artifact. Operation 604 can further include normalizing the motion value to be on a scale that corresponds with motion values obtained from other sensors in the system 10, such as from the pressure sensor 18.

Next, the method 600 includes an operation 606 of determining a location of the motion artifact. While FIG. 6 shows operation 606 occurring after completion of operation 604, in alternative examples operation 606 can occur before operation 604. Also, in some instances, operations 604, 606 may occur substantially at the same time such that they are simultaneous.

As described above, the ECG sensor 14 includes electrodes 20 attached to different locations on the body of the patient P such as the chest, right arm, left arm, right leg, left leg, and head (see FIG. 1). The location of the motion artifact is determined based on the location of the electrode 20 that detects the raw ECG signal. For example, motion artifacts detected from an electrode 20 connected to the right arm of the patient are mapped to a right arm location, motion artifacts detected from an electrode 20 connected to the left arm of the patient are mapped to a left arm location, motion artifacts detected from an electrode 20 connected to the right leg of the patient are mapped to a right leg location, motion artifacts detected from an electrode 20 connected to the left leg of the patient are mapped to a left leg location, and so on.

Additionally, raw ECG signals from a plurality of the electrodes 20 can be combined to determine a direction of the motion artifacts. For example, a right arm to left leg direction can be determined by processing the raw ECG signals from electrodes 20 attached to the right arm and left leg of the patient. Similarly, a right arm to left arm direction can be determined by processing the raw ECG signals from electrodes 20 attached to the right arm and left arm of the patient. Additional examples are possible. Thus, in some examples, motion values calculated from the raw ECG signal are vectors that indicate a direction of patient motion.

Next, the method 600 proceeds to an operation 608 of communicating the motion artifact and its location to the alarm management application 130. The motion artifact communicated in operation 608 can be used as an input for an algorithm performed by the alarm management application 130. Also, the motion artifact communicated in operation 608 can be used to determine a dynamic ranking of redundant physiological variables.

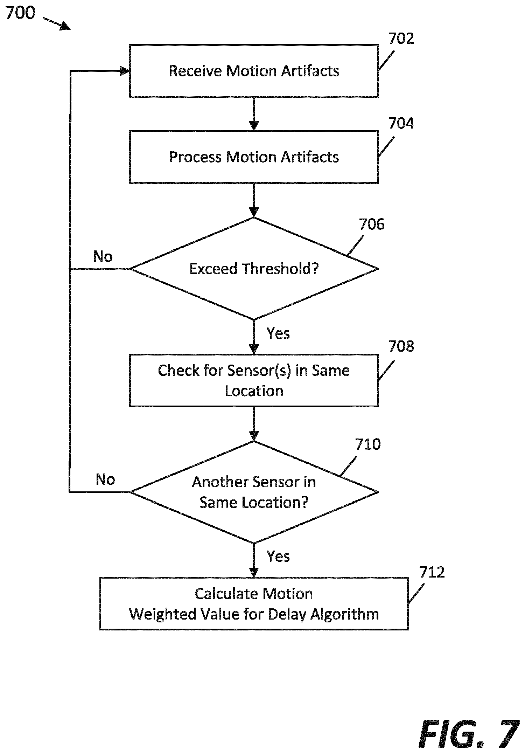

FIG. 7 illustrates an example of a method 700 of managing alarms performed by the alarm management application 130 when installed on the monitoring device 22. The method 700 can be performed to delay an alarm when patient motion is detected.

The method 700 includes an operation 702 of receiving motion artifacts from the sensors in the system 10. As described above, the motion artifacts are determined from raw data acquired from the sensors in the system 10 such as the pressure sensor 18 or ECG sensor 14 that are used to measure physiological variables (e.g., blood pressure, electrocardiogram readings), such that the sensors are used as both physiological and motion sensors. This can eliminate the need for a dedicated motion sensor, and thus reduce the number of sensors in the system 10.

In some examples, the method 700 includes an operation 704 of pre-processing the raw motion artifacts from each sensor to determine motion values. As an illustrative example, operation 704 can include performing the following algorithm:

$$\text{(i)} \qquad \text{motion\_r} = \text{process (r)}$$

where motion_r is a motion value, and r is a raw motion artifact collected from a sensor. Thus, the motion values can be calculated independent of the sensor source such as the pressure sensor 18 or ECG sensor 14. When processing the raw motion artifacts from the ECG sensor 14, the motion values can be calculated as vectors to indicate a direction of motion. A location can be assigned to each motion value such as to indicate whether it is from the right or left arm.

In other examples, the method 700 receives pre-processed motion values in operation 702 such that operation 704 does not need to be performed in the method 700. Instead, the motion artifacts are already pre-processed such as by performance of operation 412 in the method 400, operation 510 in the method 500, and/or operation 604 in the method 600.

Next, the method 700 can include an operation 706 of determining whether the motion value exceeds a predetermined threshold. Operation 706 is performed so that only motion values strong enough to affect or influence the readings from another sensor in the system 10 are considered for adjusting an alarm setting. When the motion value does not exceed the predetermined threshold (i.e., "No" at operation 706), the motion value can be ignored, and the method 700 returns to operation 702 and continues to receive motion artifacts.

When the motion value exceeds the predetermined threshold (i.e., "Yes" at operation 706), the method 700 proceeds to operation 708 of checking for sensors that measures physiological variables in the same location where the motion value is detected. When no other sensor is in the same location of the determined motion value (i.e., "No" at operation 710), the method 700 returns to operation 702 and continues receiving motion artifacts.

When at least one other physiological sensor is determined to be in the same location of the detected motion value (i.e., "Yes" at operation 710), the method 700 proceeds to an operation 712 of calculating motion weighted values for physiological measurements that are received from a sensor that is in the same location. For example, when a motion value is detected on the left arm of the patient P due to signal noise from a raw ECG signal acquired from an electrode 20 attached to the left arm of the patient P, operation 710 determines whether there are any additional physiological sensors attached to the left arm of the patient P.

In the example shown in FIG. 1, the cuff 12 and pressure sensor 18 (which are used to measure the systolic and diastolic blood pressure of the patient P) are also attached to the left arm of the patient P. Also, the SpO2 sensor 24 (which is used to measure the blood oxygen saturation of the patient P) is also attached to the left arm of the patient P.

The motion weighted values are calculated from the motion values, and are used by an alarm delay algorithm is performed to delay an alarm when abnormal physiological measurements are detected. For example, when an abnormal SpO2 measurement is detected, the alarm delay algorithm delays triggering an alarm for a predetermined period of time (e.g., 10 seconds) to determine whether the SpO2 measurement returns to the normal range. As another example, the alarm delay algorithm can determine how much the abnormal SpO2 measurement is outside of the normal range, and for how long, to determine whether to trigger an alarm.

The motion weighted value influences the effect that an abnormal physiological measurement has on the decision by the alarm delay algorithm to trigger an alarm. For example, when an abnormal physiological measurement is received when a high motion value is detected, the abnormal physiological measurement is assigned a low motion weighted value. Thus, the abnormal physiological measurement will have a small influence or will be ignored by the alarm delay algorithm when determining when to trigger an alarm.

The motion weighted values that are used by the alarm delay algorithm are calculated in accordance with the following algorithm:

$$(ii) \quad weight\_m = m*normalize(motion\_r)$$

where motion_r is the motion value, and m is a multiplier. The multiplier m is defined per pairing of sensor and physiological variable alarm. For example, the multiplier m can be assigned a value of 0.01 for motion values detected from the pressure sensor 18 for use by a SpO2 alarm. As another example, the multiplier m can be assigned a value of 0.5 for motion values detected from the ECG sensor 14 for use by a SpO2 alarm.

The normalizeQ function can use any normalization algorithms (e.g., min-max, standard score). Also, depending on the distribution of the weight_m, other types of transformations can be applied, such as log transformations.

In view of the foregoing, a larger motion value (motion_r) leads to a smaller motion weighted value (weight_m) such that an abnormal physiological measurement will have a minor influence or will be ignored for determining when to trigger an alarm. A smaller motion value (motion_r) will lead to a larger motion weighted value (weight_m) for abnormal physiological measurements that are detected when there is little or no patient motion. The motion weighted values (weight_m) are then used by the alarm delay algorithm, which can be performed by the alarm management application 130 to determine when to trigger or delay an alarm.

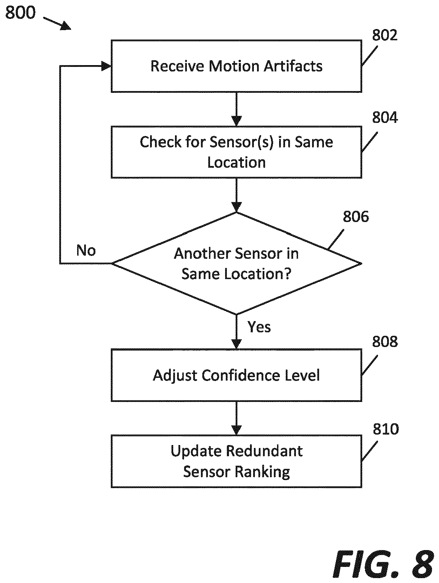

FIG. 8 illustrates an example of a method 800 of dynamic ranking of redundant physiological sensors performed by the monitoring device 22. The method 800 is performed for physiological variables that are captured by multiple sensors in the system 10. The method 800 assigns sensor confidence levels that are based on motion artifacts detected in accordance with any of the methods described above. The sensor confidence levels can be used to adjust a redundant sensor ranking to reduce inaccurate measurements used for alarm decisions. Thus, the method 800 can be used to reduce false alarms, and thereby reduce alarm fatigue.

The method 800 includes an operation 802 of receiving motion artifacts from the sensors in the system 10. As described above, the motion artifacts are determined from raw data acquired from the sensors in the system 10 (e.g., pressure sensor 18, ECG sensor 14) that are used to measure physiological variables (e.g., blood pressure, electrocardiogram readings), such that the sensors are used as both physiological and motion sensors. This can eliminate the need for a dedicated motion sensor, and thus reduce the number of sensors used by the system 10.

Next, the method 800 proceeds to operation 804 of checking for sensors that measure physiological variables in the same location of the determined motion artifact. When no other sensor is in the same location of the determined motion value (i.e., "No" at operation 806), the method 800 returns to operation 802 and continues receiving motion artifacts.

When at least one other physiological sensor is determined to be in the same location of the detected motion value (i.e., "Yes" at operation 806), the method 800 proceeds to an operation 808 of adjusting confidence levels of one or more physiological variables that are measured by sensors in the same location as the determined motion artifact.

Referring back to FIGS. 1 and 2, there are multiple sensors that can measure physiological variables redundantly. For example, heart rate can be determined from data acquired from the ECG sensor 14, SpO2 sensor 24, or contact-free sensor 26. Thus, there are at least three sets of redundant heart rate measurements: a first set determined from data acquired from the ECG sensor 14, a second set determined from data acquired from the SpO2 sensor 24, and a third set determined from data acquired from the contact-free sensor 26.

As another example, the respiration rate of the patient P can be determined from data acquired from the etO2 sensor 28 and from data acquired the contact-free sensor 26. Thus, there are two sets of redundant respiration rate measurements: a first set determined from the etO2 sensor 28 data, and a second set determined from the contact-free sensor 26 data.

In some examples, each sensor has a default confidence level such that certain sensors are preferred over others for measuring certain physiological variables, when there are redundant sensors that can measure the same physiological variable. For example, the ECG sensor 14 may be preferred for measuring the heart rate over other sensors such as the SpO2 sensor 24, which can also be used to measure heart. Alternatively, in some instances, the SpO2 sensor 24 may be preferred for measuring the heart rate over other sensors such as the ECG sensor 14.

In operation 808, the method 800 lowers the confidence level for sensors that measure physiological variables in the same location as the determined motion artifact. As an illustrative example, when a motion artifact is determined to be on the left arm of the patient (e.g., from raw ECG signals that are collected from an electrode 20 attached to the left arm of the patient), and the SpO2 sensor 24 is also attached to the left arm of the patient (see FIG. 1), the data acquired from the SpO2 sensor 24 is given a lower confidence level because of potential influence by the detected motion artifact, such that it may be inaccurate and cause a false alarm.

Next, the method 800 includes an operation 810 of updating a redundant sensor ranking for physiological variables captured by multiple sensors. In example described above where the heart rate measurements from the SpO2 sensor 24 are given a lower confidence level because the SpO2 sensor 24 is located where a motion artifact is detected, the SpO2 sensor 24 is given a lower ranking than the other sensors that can be used to measure heart rate that are not affected by the motion artifact such as the ECG sensor 14 which uses an electrode attached to the chest of the patient P to measure heart rate, or the contact-free sensor 26 which can continuously monitor the heart rate without physically contacting the patient.

The redundant sensor ranking can be used to select certain sensors over other sensors to measure the physiological variables. For example, the monitoring device 22 can select the ECG sensor 14 or the contact-free sensor 26 to measure the heart rate of the patient over the SpO2 sensor 24 because the ECG sensor 14 and the contact-free sensor 26 each have a higher ranking than the SpO2 sensor 24, based on the location of the motion artifact.

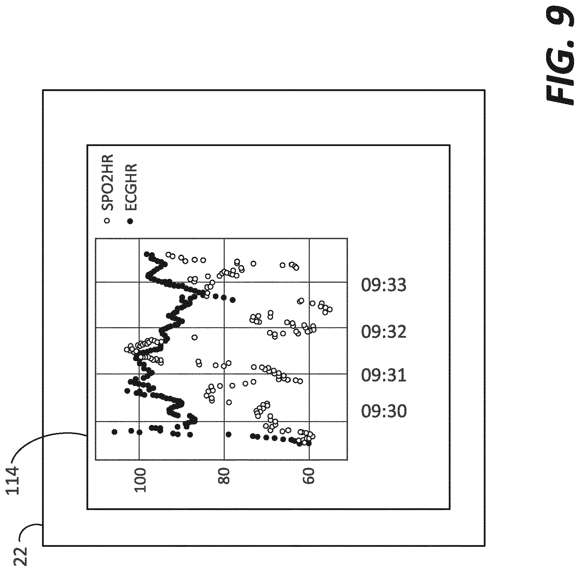

FIG. 9 illustrates an example of redundant physiological variables displayed on the display device 114 of the monitoring device 22. In this example, heart rate measurements from the SpO2 sensor 24 have a larger variance than the heart rate measurements from the ECG sensor 14. This can result from motion artifacts having a stronger influence on the date acquired from the SpO2 sensor 24, than on the data acquired from the ECG sensor 14. Thus, in this example, the heart rate measurements from the SpO2 sensor 24 are assigned a lower confidence level than the heart rate measurements from the ECG sensor 14. As a result, the ECG sensor 14 can be assigned a higher redundant sensor ranking than the SpO2 sensor 24, such that the ECG sensor 14 is selected over the SpO2 sensor 24 for measuring heart rate.

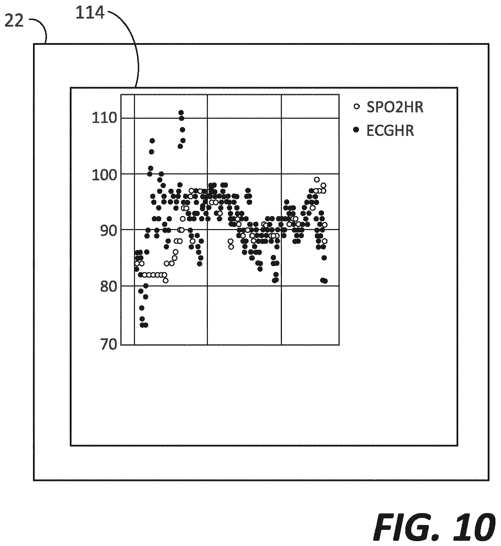

FIG. 10 illustrates another example of redundant physiological variables displayed on the display device 114 of the monitoring device 22. In this example, heart rate measurements from the ECG sensor 14 have a larger variance than the heart rate measurements from the SpO2 sensor 24. This can result from motion artifacts having a stronger influence on the date acquired from the ECG sensor 14, than on the data acquired from the SpO2 sensor 24. Thus, in this example, the heart rate measurements from the ECG sensor 14 are assigned a lower confidence level than the heart rate measurements from the SpO2 sensor 24. As a result, the SpO2 sensor 24 can be assigned a higher redundant sensor ranking than the ECG sensor 14, such that the SpO2 sensor 24 is selected over the ECG sensor 14 for measuring heart rate.

**Claims**

1. A patient monitoring device (22), comprising:

    at least one processing device (122); and
    a memory device (124) storing instructions which, when executed by the at least one processing device (122), cause the at least one processing device (122) to:

       initiate an interval mode (302) that includes measuring blood pressure at predetermined intervals over a predetermined period of time;
       take a first blood pressure measurement (304) at one of the predetermined intervals;
       determine whether the first blood pressure measurement is abnormal (306);
       take a second blood pressure measurement (308) after a predetermined delay when the first blood pressure measurement is determined abnormal;
       compare the first blood pressure measurement to the second blood pressure measurement (318) wherein if the second blood pressure measurement is within a predefined threshold of the first blood pressure measurement, the first blood pressure measurement is confirmed as abnormal; and
       trigger an alarm (312) when the first blood pressure measurement is confirmed as abnormal.

2. The patient monitoring device (22) of claim 1, wherein the instructions, when executed by the at least one processing device (122), further cause the at least one processing device (122) to:
    store at least one of the first blood pressure measurement, the second blood pressure measurement, and an average of the first and second blood pressure measurements in an electronic medical record.

3. The patient monitoring device (22) of either claim 1 or claim 2, wherein the instructions, when executed by the at least one processing device (122), further cause the at least one processing device (122) to:

    determine whether a delay limit for the one of the predetermined intervals is reached (314);
    determine an average value of the first and second blood pressure measurements when the delay limit is reached (318); and
    store the average value in an electronic medical record.

4. The patient monitoring device (22) of any preceding claim, wherein the instructions, when executed by the at least one processing device (122), further cause the at least one processing device (122) to:

> determine whether a delay limit for the one of the predetermined intervals is reached (318);
> provide an instruction to remain still when the delay limit is not reached (316); and
> take a third blood pressure measurement after the predetermined delay (308).

5. The patient monitoring device (22) of claim 4, wherein the instructions, when executed by the at least one processing device (122), further cause the at least one processing device (122) to:

> determine at least one of an average value and a majority value of the first, second, and third blood pressure measurements when the delay limit is reached (318); and
> store in an electronic medical record the average value or the majority value.

6. The patient monitoring device (22) of either claim 4 or claim 5, wherein the delay limit is one of: a default value; and a value set by a clinician.

7. The patient monitoring device (22) of any preceding claim, wherein the instructions, when executed by the at least one processing device (122), further cause the at least one processing device (122) to:

> inflate a cuff (406) around a limb of a patient to have a partial pressure less than a pressure applied by the cuff to the limb when measuring the blood pressure in the interval mode;
> monitor an internal pressure inside the cuff (408);
> identify motion artifacts (412) based on changes in the internal pressure; and
> delay an alarm (414) triggered by a physiological sensor based on the motion artifacts.

8. The patient monitoring device (22) of claim 7, wherein the instructions, when executed by the at least one processing device (122), further cause the at least one processing device (122) to:

> check a status of the cuff (402) during the interval mode for measuring the blood pressure; and
> inflate the cuff (406) to have the partial pressure when the status of the cuff indicates the cuff is not being used to measure the blood pressure in the interval mode.

9. The patient monitoring device (22) of either claim 7 or claim 8, wherein the instructions, when executed by the at least one processing device (122), further cause the at least one processing device (122) to:

> receive an abnormal measurement (502) from the physiological sensor; and
> inflate the cuff (504) to have the partial pressure in response to the abnormal measurement.

10. The patient monitoring device (22) of any one of claims 7 to 9, wherein the instructions, when executed by the at least one processing device (122), further cause the at least one processing device (122) to:

> assign a location to the motion artifacts based on the location of the cuff (12);
> identify one or more sensors taking physiological measurements in the location assigned to the motion artifacts (708);
> calculate motion weighted values (712) based on the motion artifacts for the physiological measurements taken by the one or more sensors in the location assigned to the motion artifacts; and
> use the motion weighted values in an alarm delay algorithm.

11. The patient monitoring device (22) of any one of claims 7 to 10, wherein the instructions, when executed by the at least one processing device (122), further cause the at least one processing device (122) to:

> receive electrocardiogram signals (602) from an electrode attached to a body;
> identify motion artifacts (604) from the electrocardiogram signals;
> assign a location to the motion artifacts (606) based on where the electrode is attached to the body;
> identify sensors (708) taking physiological measurements in the location of the motion artifacts;
> calculate motion weighted values (712) based on the motion artifacts for the physiological measurements taken by the sensors; and

use the motion weighted values in an alarm delay algorithm.

12. The patient monitoring device (22) of claim 11, wherein the instructions, when executed by the at least one processing device (122), further cause the at least one processing device (122) to:

receive the electrocardiogram signals (602) from multiple electrodes;
determine a direction of the motion artifacts (604) using the electrocardiogram signals; and
calculate the motion weighted values (608) as vectors.

13. The patient monitoring device (22) of either claim 11 or claim 12, wherein the motion artifacts are determined from signal noise in the electrocardiogram signals, and the electrocardiogram signals are filtered to exclude high frequency noise.

14. The patient monitoring device (22) of any one of claims 11 to 13, wherein the instructions, when executed by the at least one processing device (122), further cause the at least one processing device (122) to:

identify motion artifacts (510) from an inflatable cuff used for measuring blood pressure;
use the motion artifacts from the inflatable cuff to calculate motion weighted values (712); and
normalize the motion weighted values calculated from the electrocardiogram signals to be on a common scale with the motion weighted values calculated from the inflatable cuff.

**Patentansprüche**

1. Patientenüberwachungsvorrichtung (22), die aufweist:

wenigstens eine Verarbeitungsvorrichtung (122); und
eine Speichervorrichtung (124), die Anweisungen speichert, die bei Ausführung durch die wenigstens eine Verarbeitungsvorrichtung (122) die wenigstens eine Verarbeitungsvorrichtung (122) veranlassen zum:

Einleiten eines Intervallmodus (302), der das Messen von Blutdruck in vorbestimmten Intervallen über eine vorbestimmte Zeitspanne umfasst,
Messen eines ersten Blutdruckmesswerts (304) in einem der vorbestimmten Intervalle;
Bestimmen, ob der erste Blutdruckmesswert abnormal ist (306) ;
Messen eines zweiten Blutdruckmesswerts (308) nach einer vorbestimmten Verzögerung, wenn der erste Blutdruckmesswert als abnormal bestimmt wird;
Vergleichen des ersten Blutdruckmesswerts mit dem zweiten Blutdruckmesswert (318), wobei, wenn der zweite Blutdruckmesswert innerhalb einer vordefinierten Schwelle des ersten Blutdruckmesswerts liegt, der erste Blutdruckmesswert als abnormal bestätigt wird; und
Auslösen eines Alarms (312), wenn der erste Blutdruckmesswert als abnormal bestätigt wird.

2. Patientenüberwachungsvorrichtung (22) nach Anspruch 1, wobei die Anweisungen bei Ausführung durch die wenigstens eine Verarbeitungsvorrichtung (122) ferner die wenigstens eine Verarbeitungsvorrichtung (122) veranlassen zum:
Speichern wenigstens eines von dem ersten Blutdruckmesswert, dem zweiten Blutdruckmesswert und einem Durchschnitt des ersten und des zweiten Blutdruckmesswerts in einer elektronischen medizinischen Akte.

3. Patientenüberwachungsvorrichtung (22) nach Anspruch 1 oder Anspruch 2, wobei die Anweisungen bei Ausführung durch die wenigstens eine Verarbeitungsvorrichtung (122) ferner die wenigstens eine Verarbeitungsvorrichtung (122) veranlassen zum:

Bestimmen, ob eine Verzögerungsgrenze für das eine der vorbestimmten Intervalle erreicht wird, (314);
Bestimmen eines Durchschnittswerts der ersten und zweiten Blutdruckmesswerte, wenn die Verzögerungsgrenze erreicht wird, (318); und
Speichern des Durchschnittswerts in einer elektronischen medizinischen Akte.

4. Patientenüberwachungsvorrichtung (22) nach einem der vorhergehenden Ansprüche, wobei die Anweisungen bei Ausführung durch die wenigstens eine Verarbeitungsvorrichtung (122) ferner die wenigstens eine Verarbeitungsvorrichtung (122) veranlassen zum:

> Bestimmen, ob eine Verzögerungsgrenze für das eine der vorbestimmten Intervalle erreicht wird, (318);
> Geben einer Anweisung zum Stillhalten, wenn die Verzögerungsgrenze nicht erreicht wird, (316); und
> Messen eines dritten Blutdruckmesswerts nach der vorbestimmten Verzögerung (308).

**5.** Patientenüberwachungsvorrichtung (22) nach Anspruch 4, wobei die Anweisungen bei Ausführung durch die wenigstens eine Verarbeitungsvorrichtung (122) ferner die wenigstens eine Verarbeitungsvorrichtung (122) veranlassen zum:

> Bestimmen von wenigstens einem von einem Durchschnittswert und einem Mehrheitswert des ersten, zweiten und dritten Blutdruckmesswerts, wenn die Verzögerungsgrenze erreicht wird, (318); und
> Speichern des Durchschnittswerts oder des Mehrheitswerts in einer elektronischen medizinischen Akte.

**6.** Patientenüberwachungsvorrichtung (22) nach Anspruch 4 oder Anspruch 5, wobei die Verzögerungsgrenze eine der folgenden ist: ein Vorgabewert; und ein von einer medizinischen Fachperson gesetzter Wert.

**7.** Patientenüberwachungsvorrichtung (22) nach einem der vorhergehenden Ansprüche, wobei die Anweisungen bei Ausführung durch die wenigstens eine Verarbeitungsvorrichtung (122) ferner die wenigstens eine Verarbeitungsvorrichtung (122) veranlassen zum:

> Aufblasen der Manschette (406) um ein Körperglied, so dass sie einen Partialdruck aufweist, der kleiner als ein beim Messen des Blutdrucks im Intervallmodus durch die Manschette auf das Körperglied ausgeübter Druck ist;
> Überwachen eines Innendrucks im Inneren der Manschette (408) ;
> Identifizieren von Bewegungsartefakten (412) auf Basis von Änderungen des Innendrucks; und
> Verzögern eines durch einen physiologischen Sensor ausgeübten Alarms (414) auf Basis der Bewegungsartefakte.

**8.** Patientenüberwachungsvorrichtung (22) nach Anspruch 7, wobei die Anweisungen bei Ausführung durch die wenigstens eine Verarbeitungsvorrichtung (122) ferner die wenigstens eine Verarbeitungsvorrichtung (122) veranlassen zum:

> Prüfen eines Status der Manschette (402) während des Intervallmodus zum Messen des Blutdrucks; und
> Aufblasen der Manschette (406), so dass sie einen Partialdruck aufweist, wenn der Status der Manschette anzeigt, dass die Manschette nicht zum Messen des Blutdrucks im Intervallmodus verwendet wird.

**9.** Patientenüberwachungsvorrichtung (22) nach Anspruch 7 oder Anspruch 8, wobei die Anweisungen bei Ausführung durch die wenigstens eine Verarbeitungsvorrichtung (122) ferner die wenigstens eine Verarbeitungsvorrichtung (122) veranlassen zum:

> Empfangen eines abnormalen Messwerts (502) von dem physiologischen Sensor; und
> Aufblasen der Manschette (504), so dass sie den Partialdruck aufweist, als Reaktion auf den abnormalen Messwert.

**10.** Patientenüberwachungsvorrichtung (22) nach einem der Ansprüche 7 bis 9, wobei die Anweisungen bei Ausführung durch die wenigstens eine Verarbeitungsvorrichtung (122) ferner die wenigstens eine Verarbeitungsvorrichtung (122) veranlassen zum:

> Zuweisen einer Position zu den Bewegungsartefakten auf Basis der Position der Manschette (12);
> Identifizieren von einem oder mehreren Sensoren, die an der den Bewegungsartefakten zugewiesenen Position physiologische Messwerte messen, (708);
> Berechnen von bewegungsgewichteten Werten (712) auf Basis der Bewegungsartefakte für die durch den einen oder die mehreren Sensoren an der den Bewegungsartefakten zugewiesenen Position gemessenen physiologischen Messwerte; und
> Verwenden der bewegungsgewichteten Werte in einem Alarmverzögerungsalgorithmus.

**11.** Patientenüberwachungsvorrichtung (22) nach einem der Ansprüche 7 bis 10, wobei die Anweisungen bei Ausführung durch die wenigstens eine Verarbeitungsvorrichtung (122) ferner die wenigstens eine Verarbeitungsvorrichtung (122) veranlassen zum:

Empfangen von Elektrokardiogrammsignalen (602) von einer an einem Körper angebrachten Elektrode;
Identifizieren von Bewegungsartefakten (604) von den Elektrokardiogrammsignalen;
Zuweisen einer Position zu den Bewegungsartefakten (606) auf Basis der Anbringungsstelle der Elektrode am Körper;
Identifizieren von Sensoren (708), die physiologische Messwerte an der Position der Bewegungsartefakte messen;
Berechnen von bewegungsgewichteten Werten (712) auf Basis der Bewegungsartefakte für die von den Sensoren gemessenen physiologischen Messwerte; und
Verwenden der bewegungsgewichteten Werte in einem Alarmverzögerungsalgorithmus.

12. Patientenüberwachungsvorrichtung (22) nach Anspruch 11, wobei die Anweisungen bei Ausführung durch die wenigstens eine Verarbeitungsvorrichtung (122) ferner die wenigstens eine Verarbeitungsvorrichtung (122) veranlassen zum:

Empfangen der Elektrokardiogrammsignale (602) von mehreren Elektroden;
Bestimmen einer Richtung der Bewegungsartefakte (604) unter Verwendung der Elektrokardiogrammsignale; und
Berechnen der bewegungsgewichteten Werte (608) als Vektoren.

13. Patientenüberwachungsvorrichtung (22) nach Anspruch 11 oder Anspruch 12, wobei die Bewegungsartefakte anhand von Signalrauschen in Elektrokardiogrammsignalen bestimmt werden und die Elektrokardiogrammsignale zum Ausschließen von hochfrequentem Rauschen gefiltert werden.

14. Patientenüberwachungsvorrichtung (22) nach einem der Ansprüche 11 bis 13, wobei die Anweisungen bei Ausführung durch die wenigstens eine Verarbeitungsvorrichtung (122) ferner die wenigstens eine Verarbeitungsvorrichtung (122) veranlassen zum:

Identifizieren von Bewegungsartefakten (510) von einer zum Messen von Blutdruck verwendeten aufblasbaren Manschette;
Verwenden der Bewegungsartefakte von der aufblasbaren Manschette zum Berechnen von bewegungsgewichteten Werten (712); und
Normalisieren der anhand der Elektrokardiogrammsignale berechneten bewegungsgewichteten Werte, so dass sie auf einem gemeinsamen Maßstab mit den anhand der aufblasbaren Manschette berechneten bewegungsgewichteten Werte liegen.

**Revendications**

1. Dispositif de surveillance de patient (22), comprenant :

au moins un dispositif de traitement (122) ; et
un dispositif de mémoire (124) stockant des instructions qui, lorsque exécutées par au moins un dispositif de traitement (122), font que le au moins un dispositif de traitement (122) :

lance un mode d'intervalles (302) qui comprend mesurer une pression artérielle à des intervalles prédéterminés au cours d'une période de temps prédéterminée ;
prend une première mesure de pression artérielle (304) à l'un des intervalles prédéterminés ;
détermine si la première mesure de pression artérielle est anormale (306) ;
prend une deuxième mesure de pression artérielle (308) après un délai prédéterminé lorsque la première mesure de pression artérielle est déterminée anormale ;
compare la première mesure de pression artérielle à la deuxième mesure de pression artérielle (318), dans lequel si la deuxième mesure de pression artérielle est dans un seuil prédéfini de la première mesure de pression artérielle, la première mesure de pression artérielle est confirmée comme anormale ; et
déclenche une alarme (312) lorsque la première mesure de pression artérielle est confirmée comme anormale.

**2.** Dispositif de surveillance de patient (22) selon la revendication 1, dans lequel les instructions, lorsque exécutées par le au moins un dispositif de traitement (122), font en outre que le au moins un dispositif de traitement (122) :
stocke au moins l'une d'entre la première mesure de pression artérielle, la deuxième mesure de pression artérielle et une moyenne de la première et de la deuxième mesure de pression artérielle dans un dossier médical électronique.

**3.** Dispositif de surveillance de patient (22) selon la revendication 1 ou la revendication 2, dans lequel les instructions, lorsque exécutées par le au moins un dispositif de traitement (122), font en outre que le au moins un dispositif de traitement (122) :

déterminé si un délai-limite pour l'un des intervalles prédéterminés est atteint (314) ;
déterminé une valeur moyenne de la première et de la deuxième mesure de pression artérielle lorsque le délai-limite est atteint (318) ; et
stocke la valeur moyenne dans un dossier médical électronique.

**4.** Dispositif de surveillance de patient (22) selon l'une quelconque des revendications précédentes, dans lequel les instructions, lorsque exécutées par le au moins un dispositif de traitement (122), font en outre que le au moins un dispositif de traitement (122) :

déterminé si un délai-limite pour l'un des intervalles prédéterminés est atteint (318) ;
donne une instruction de rester sans bouger lorsque le délai-limite n'est pas atteint (316) ; et
prend une troisième mesure de pression artérielle après le délai prédéterminé (308).

**5.** Dispositif de surveillance de patient (22) selon la revendication 4, dans lequel les instructions, lorsque exécutées par le au moins un dispositif de traitement (122), font en outre que le au moins un dispositif de traitement (122) :

déterminé au moins l'une d'entre une valeur moyenne et une valeur majoritaire des première, deuxième et troisième mesures de pression artérielle lorsque le délai-limite est atteint (318) ; et
stocke la valeur moyenne ou la valeur majoritaire dans un dossier médical électronique.

**6.** Dispositif de surveillance de patient (22) selon la revendication 4 ou la revendication 5, dans lequel le délai-limite est l'une d'entre : une valeur par défaut ; et une valeur réglée par un clinicien.

**7.** Dispositif de surveillance de patient (22) selon l'une quelconque des revendications précédentes, dans lequel les instructions, lorsque exécutées par le au moins un dispositif de traitement (122), font en outre que le au moins un dispositif de traitement (122) :

gonfle un brassard (406) autour d'un membre du patient pour avoir une pression partielle moindre qu'une pression appliquée par le brassard sur le membre lorsque mesurant la pression artérielle dans le mode d'intervalles ;
surveille une pression interne dans le brassard (408) ;
identifie des artefacts de mouvement (412) basés sur des changements dans la pression interne ; et
retarde une alarme (414) déclenchée par un capteur physiologique sur la base des artefacts de mouvement.

**8.** Dispositif de surveillance de patient (22) selon la revendication 7, dans lequel les instructions, lorsque exécutées par le au moins un dispositif de traitement (122), font en outre que le au moins un dispositif de traitement (122) :

vérifie un état du brassard (402) pendant le mode d'intervalles pour mesurer la pression artérielle ; et
gonfle le brassard (406) pour avoir la pression partielle lorsque l'état du brassard indique que le brassard n'est pas utilisé pour mesurer la pression artérielle dans le mode d'intervalles.

**9.** Dispositif de surveillance de patient (22) selon la revendication 7 ou la revendication 8, dans lequel les instructions, lorsque exécutées par le au moins un dispositif de traitement (122), font en outre que le au moins un dispositif de traitement (122) :

reçoit une mesure anormale (502) du capteur physiologique ; et
gonfle le brassard (504) pour avoir la pression partielle en réponse à la mesure anormale.

**10.** Dispositif de surveillance de patient (22) selon l'une quelconque des revendications 7 à 9, dans lequel les instructions, lorsque exécutées par le au moins un dispositif de traitement (122), font en outre que le au moins un dispositif de traitement (122) :

attribue un emplacement aux artefacts de mouvement sur la base de l'emplacement du brassard (12) ;
identifie un ou plusieurs capteurs prenant des mesures physiologiques à l'emplacement attribué aux artefacts de mouvement (708) ;
calcule des valeurs de mouvement pondérées (712) sur la base des artefacts de mouvement pour les mesures physiologiques prises par l'un ou les plusieurs capteurs à l'emplacement attribué aux artefacts de mouvement ; et
utilise les valeurs de mouvement pondérées dans un algorithme de délai d'alarme.

11. Dispositif de surveillance de patient (22) selon l'une quelconque des revendications 7 à 10, dans lequel les instructions, lorsque exécutées par le au moins un dispositif de traitement (122), font en outre que le au moins un dispositif de traitement (122) :

reçoit des signaux d'électrocardiogramme (602) d'une électrode attachée à un corps ;
identifie des artefacts de mouvement (604) des signaux d'électrocardiogramme ;
attribue un emplacement aux artefacts de mouvement (606) sur la base d'où l'électrode est attachée au corps ;
identifie des capteurs (708) prenant des mesures physiologiques à l'emplacement des artefacts de mouvement ;
calcule des valeurs de mouvement pondérées (712) sur la base des artefacts de mouvement pour les mesures physiologiques prises par les capteurs ; et
utilise les valeurs de mouvement pondérées dans un algorithme de délai d'alarme.

12. Dispositif de surveillance de patient (22) selon la revendication 11, dans lequel les instructions, lorsque exécutées par le au moins un dispositif de traitement (122), font en outre que le au moins un dispositif de traitement (122) :

reçoit les signaux d'électrocardiogramme (602) d'électrodes multiples ;
détermine une direction des artefacts de mouvement (604) en utilisant les signaux d'électrocardiogramme ; et
calcule les valeurs de mouvement pondérées (608) comme des vecteurs.

13. Dispositif de surveillance de patient (22) selon la revendication 11 ou la revendication 12, dans lequel les artefacts de mouvement sont déterminés du bruit des signaux dans les signaux d'électrocardiogramme et les signaux d'électrocardiogramme sont filtrés pour exclure un bruit haute fréquence.

14. Dispositif de surveillance de patient (22) selon l'une quelconque des revendications 11 à 13, dans lequel les instructions, lorsque exécutées par le au moins un dispositif de traitement (122), font en outre que le au moins un dispositif de traitement (122) :

identifie des artefacts de mouvement (510) d'un brassard gonflable utilisé pour mesurer une pression artérielle ;
utilise les artefacts de mouvement du brassard gonflable pour calculer des valeurs de mouvement pondérées (712) ; et
normalise les valeurs de mouvement pondérées calculées des signaux d'électrocardiogramme pour qu'elles soient sur une échelle commune avec les valeurs de mouvement pondérées calculées du brassard gonflable.

**FIG. 1**

EP 4 147 634 B1

**FIG. 2**

300

302

Initiate Interval Mode

304

Measure Blood Pressure

306

No ← Blood Pressure Abnormal?

Yes

308

Retake Blood Pressure ← Provide Instruction

316

No

310

No ← Blood Pressure Confirmed? → No → Delay Limit Reached?

314

Yes

Yes

Perform Action ← Calculate Average Value or Majority Value

312

318

**FIG. 3**

400

FIG. 4

500

502
Receive Abnormal Reading

504
Inflate Cuff to Partial Pressure

506
Monitor Pressure Inside Cuff

508
Pressure Change Detected?

No

Yes

510
Determine Motion Artifact

512
Communicate Motion Artifact

*FIG. 5*

600

602
Receive Raw ECG Signal

604
Process Raw ECG Signal to
Determine Motion Artifact

606
Determine Location of
Motion Artifact

608
Communicate Motion Artifact
and Location

**FIG. 6**

700

Receive Motion Artifacts 702

Process Motion Artifacts 704

Exceed Threshold? 706

No

Yes

Check for Sensor(s) in Same Location 708

Another Sensor in Same Location? 710

No

Yes

Calculate Motion Weighted Value for Delay Algorithm 712

*FIG. 7*

800

802

Receive Motion Artifacts

804

Check for Sensor(s) in Same
Location

806

No

Another Sensor in
Same Location?

Yes

808

Adjust Confidence Level

810

Update Redundant
Sensor Ranking

*FIG. 8*

**FIG. 9**

EP 4 147 634 B1

○ SPO2HR
● ECGHR

110
100
90
80
70

114

22

*FIG. 10*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4050452 A1
- US 4417587 A1
- US 9775758 B